Europäisches Patentamt

European Patent Office

Office européen des brevets

(11) Veröffentlichungsnummer: **0 436 088 A1**

## (12) EUROPÄISCHE PATENTANMELDUNG

(21) Anmeldenummer: 90121362.9

(22) Anmeldetag: 08.11.90

(51) Int. Cl.5: **C07D 241/28**

(30) Priorität: 05.01.90 DE 4000168

(43) Veröffentlichungstag der Anmeldung:
10.07.91 Patentblatt 91/28

(84) Benannte Vertragsstaaten:
**CH DE ES FR GB IT LI NL**

(71) Anmelder: **HÜLS AKTIENGESELLSCHAFT**
**Patentabteilung / PB 15 - Postfach 13 20**
**W-4370 Marl 1(DE)**

(72) Erfinder: **Feld, Marcel, Dr.**
**Im Lochgarten 56**
**W-5000 Köln 90(DE)**
Erfinder: **Vogt, Wilhelm, Dr.**
**Kleiststrasse 39**
**W-5000 Köln 40(DE)**
Erfinder: **Hunds, Artur, Dr.**
**Von-Weichs-Strasse 1**
**W-5300 Bonn 1(DE)**

(54) **Verfahren zur Herstellung von 3-Aminopyrazin-2-carbonsäure und den entsprechenden Alkalisalzen.**

(57) Zur Herstellung von 3-Aminopyrazin-2-carbonsäure oder deren Alkalisalzen wird vorgeschlagen, 2-Amino-4-hydroxypteridin (Pterin) bei 140 bis 220 °C mit weniger als 4, vorzugsweise 2 bis 3 Mol Alkalihydroxid je Mol Pterin umzusetzen und durch Ansäuern als Carbonsäure zu fällen bzw. durch Kristallisieren die Alkalisalze zu gewinnen. Sehr bevorzugt wird das nicht umgesetzte Pterin mit einem Carbonsäureester, sehr bevorzugt Essigsäuremethyl- oder ethylester, in der Reaktionslösung oder in einem Reinigungsschritt bei pH 3 bis 10 gefällt und abgetrennt.

EP 0 436 088 A1

## VERFAHREN ZUR HERSTELLUNG VON 3-AMINOPYRAZIN-2-CARBONSÄURE UND DEN ENTSPRECHENDEN ALKALISALZEN

Die vorliegende Erfindung betrifft die Herstellung von 3-Aminopyrazin-2-carbonsäure und der entsprechenden Alkalisalze, die als Zwischenprodukte insbesondere für die Synthese pharmazeutischer Wirkstoffe, beispielsweise des Diureticums Amilorid, von Interesse sind.

Es ist bekannt, 3-Aminopyrazin-2-carbonsäure durch Hydrolyse von Lumazin (2,4-Dihydroxypteridin) mit Natronlauge und Ansäuern der dabei erhaltenen wäßrigen Lösung des Natriumsalzes der 3-Aminopyrazin-2-carbonsäure herzustellen (J. Amer. Chem. Soc. 67 (1945), 802). Das Lumazin wird dafür in bekannter Weise durch Glyoxal-Addition an das allerdings nur durch eine vielstufige Synthese erhältliche und somit entsprechend teure 5,6-Diamino-2,4-dihydroxypyrimidin gewonnen.

Ein unter wirtschaftlichen Gesichtspunkten günstigerer Ausgangsstoff könnte Pterin (2-Amino-4-hydroxypteridin) sein, das in ebenfalls bekannter Weise durch Glyoxal-Addition an das in Form des Sulfates in technischem Maße hergestellte 2,4,5-Triamino-6-hydroxypyrimidin erhalten werden kann.

Es bestand somit die Aufgabe, ein Verfahren zur Spaltung von Pterin zu 3-Aminopyrazin-2-carbonsäure bzw. deren Alkalisalzen zu finden. Diese Aufgabe wird durch das erfindungsgemäße Verfahren gelöst.

Gegenstand der Erfindung ist daher das Verfahren nach dem Patentanspruch und den Unteransprüchen.

Nach dem erfindungsgemäßen Verfahren wird Pterin in wäßrigem Medium mit Natronlauge und/oder Kalilauge bei Temperaturen von 140 bis 220 °C, vorzugsweise bei 150 bis 170 °C, zu den Alkalisalzen der 3-Aminopyrazin-2-carbonsäure gespalten. Aus den wäßrigen Lösungen oder Suspensionen der Salze kann mittels Mineralsäuren auf bekannte Weise die schwerlösliche 3-Aminopyrazin-2-carbonsäure gefällt und durch eine übliche Fest-Flüssig-Trennung abgetrennt werden. Bei geeigneter Wahl der Konzentrationsverhältnisse können aus den wäßrig-alkalischen Reaktionslösungen erfindungsgemäß aber auch die Alkalisalze, vorzugsweise das Kaliumsalz der 3-Aminopyrazin-2-carbonsäure in kristalliner Form abgetrennt werden.

Die erfindungsgemäße Gewinnung von 3-Aminopyrazin-2-carbonsäure in befriedigender Ausbeute und Reinheit durch alkalische Spaltung des Pterins ist dadurch sehr erschwert, daß unter den Bedingungen dieser Hydrolyse auch die aus der Literatur bekannte Substitution der NH$_2$-Gruppe des Zielproduktes durch eine OH-Gruppe erfolgt. Sowohl die gewünschte Pterin-Spaltung als auch die unerwünschte Substitutionsreaktion werden jeweils durch hohe Alkalikonzentrationen, hohe Temperaturen und lange Reaktionszeiten begünstigt. Es sind daher spezielle Reaktionsbedingungen erforderlich, um einerseits das in Wasser nahezu unlösliche Pterin weitestgehend umzusetzen, andererseits jedoch die Folgereaktion bestmöglich zu unterdrücken. Die Aufgabe ist noch dadurch erschwert, daß naturgemäß die Geschwindigkeit der Pterin-Spaltung mit fortschreitender Reaktion durch Verringerung der Pterin-Konzentration abnimmt, während umgekehrt die Geschwindigkeit der unerwünschten Folgereaktion mit steigender Konzentration zunimmt.

Trotz dieser Schwierigkeiten können nach dem erfindungsgemäßen Verfahren 3-Aminopyrazin-2-carbonsäure bzw. die entsprechenden Alkalisalze in guten Ausbeuten und mit teilweise sehr hohem Reinheitsgrad gewonnen werden.

Im Hinblick auf das Ziel einer befriedigenden Produktausbeute mußte bei hohem Pterin-Umsatz insbesondere die erwähnte Substitution der NH$_2$-Gruppe des Zielproduktes weitestgehend vermieden werden. Als besonders nachteilig für eine hohe Ausbeute an Zielprodukt hat sich ein größerer Alkaliüberschuß erwiesen. Erfindungsgemäß werden daher weniger als 4 Mol Alkalihydroxid pro Mol Pterin eingesetzt. Bevorzugt werden 2 bis 3 Mol Alkalihydroxid pro Mol Pterin verwendet.

Das Alkali kann dabei zusammen mit dem Pterin vorgelegt oder ganz oder teilweise erst unter den Reaktionsbedingungen portionsweise oder kontinuierlich, vorzugsweise in Form einer wäßrigen Lösung zugesetzt werden. Unter Berücksichtigung der Löslichkeitsverhältnisse ist es allerdings sinnvoll, zumindest eine bezogen auf das Pterin äquimolare Teilmenge des Alkalis bereits mit dem Pterin zusammen vorzulegen.

Neben dem Alkali/Pterin-Verhältnis kommt auch der Reaktionstemperatur bei der alkalischen Pterin-Spaltung eine große Bedeutung für das Erreichen befriedigender Ausbeuten zu. Es wurde gefunden, daß die Geschwindigkeit der Pterin-Spaltung bei Temperaturen unter 140 °C für ein technisch nutzbares Verfahren zu gering ist. Höhere Temperaturen begünstigen andererseits nicht nur die gewünschte Reaktion, sondern auch die unerwünschte Folgereaktion. Aus diesem Grund sind Temperaturen von über 200 °C, insbesondere von über 220 °C weniger geeignet, zumal auch mit zunehmender Temperatur die Ansprüche an die Korrosionsfestigkeit des Reaktormaterials steigen. Die erfindungsgemäße Pterin-Spaltung erfolgt dementsprechend bei Temperaturen von 140 bis 220 °C, vorzugsweise bei 150 bis 170 °C.

Die Wassermenge des Einsatzgemisches kann beim erfindungsgemäßen Verfahren prinzipiell in weiten Grenzen variiert werden. Bei der Isolierung des Zielproduktes als 3-Aminopyrazin-2-carbonsäure aus dem Reaktionsgemisch mittels einer Säure wirkt sich ein mehr oder weniger starker Verdünnungsgrad auch nicht sehr gravierend auf die Ausbeute aus. Dies ist im Falle der in Wasser sehr gut löslichen Alkalisalze der 3-Aminopyrazin-2-carbonsäure allerdings anders. Zur Verringerung der Lösungsverluste wird hier das Zielprodukt aus einem Reaktionsgemisch isoliert, welches das Zielprodukt in möglichst hoher Konzentration enthält. Die Wassermenge muß allerdings ausreichen, um die zusammen mit dem Zielprodukt entstandenen anorganischen Alkali- und Ammoniumsalze in Lösung zu halten.

Die Einstellung der vorteilhaft hohen Salzkonzentration im Falle der direkten Gewinnung des Alkalisalzes der 3-Aminopyrazin-2-carbonsäure kann durch Aufkonzentrieren der Reaktionslösung nach oder auch während der Reaktion erfolgen. Im Hinblick auf eine erstrebenswert hohe Raum-Zeit-Ausbeute ist es allerdings sinnvoll, bereits im Einsatzgemisch eine möglichst geringe Wassermenge vorzugeben. Vorzugsweise werden beim erfindungsgemäßen Verfahren daher pro Gew.-Teil Pterin 1,5 bis 3 Gew.-Teile Wasser eingesetzt, insbesondere im Falle der direkten Gewinnung des Kaliumsalzes der 3-Aminopyrazin-2-carbonsäure.

Im Falle der direkten Gewinnung des Kaliumsalzes der 3-Aminopyrazin-2-carbonsäure kann die Kaliumionenkonzentration der heißen Reaktionslösung auch nachträglich noch, vor der Kristallisation des Zielproduktes, beispielsweise durch Zugabe von Kaliumhydroxid, erhöht werden, um so die Lösungsverluste des Zielproduktes zu vermindern. Es ist in diesem Falle auch angebracht, zum Auswaschen des Filterkuchens ein Kaliumionen enthaltendes Wasser und/oder einen wäßrig-verdünnten Alkohol zu verwenden. So kann beispielsweise zur weitgehenden Verdrängung der intensiv gefärbten Mutterlauge zunächst mit einer 25 bis 35 Gew.-%igen wäßrigen $K_2CO_3$-Lösung und anschließend mit 55 bis 65 Gew.-%igem wäßrigen Methanol gewaschen werden.

Die Gewinnung des Zielproduktes in Form des Alkalisalzes der 3-Aminopyrazin-2-carbonsäure, vorzugsweise des Kaliumsalzes, hat den Vorteil, daß dabei die durch unerwünschte Folgereaktion gebildete Hydroxypyrazincarbonsäure als Alkalisalz in Lösung bleibt und mit der Mutterlauge abgetrennt wird. Das Kaliumsalz der 3-Aminopyrazin-2-carbonsäure kristallisiert beim Abkühlen der Reaktionslösung mit 1 Mol Kristallwasser aus, das es jedoch bei Temperaturen von über 100 °C verliert.

Zur Reinigung kann das in Form der 3-Aminopyrazin-2-carbonsäure oder besonders des entsprechenden Alkalisalzes gewonnene Rohprodukt einer Reinigungsoperation unterworfen werden, bei der nicht umgesetztes Pterin nach Einstellung eines pH-Wertes von 3 bis 10 selektiv abgetrennt wird. Nach einer bevorzugten Variante des erfindungsgemäßen Verfahrens erfolgt die selektive Fällung und Abtrennung des nicht umgesetzten Pterins jedoch direkt aus der Reaktionslösung vor der Fällung der 3-Aminopyrazin-2-carbonsäure bzw. vor dem Auskristallisieren des entsprechenden Alkalisalzes. Dazu wird die Reaktionslösung bei erhöhten Temperaturen, die ausreichen, um das Alkalisalz der 3-Aminopyrazin-2-carbonsäure in Lösung zu halten, auf einen pH-Wert von kleiner 10 und größer 3 gebracht. Bevorzugt wird dazu eine Carbonsäure bzw. gemäß DE-PS 37 23 874 ein Carbonsäureester, sehr bevorzugt ein Alkylester der Essigsäure verwendet. Geeignete Temperaturen liegen zwischen 30 °C und dem Siedepunkt des Reaktionsgemisches.

Nach einer derartigen selektiven Fällung des nicht umgesetzten Pterins und dessen Abtrennung durch eine übliche Fest-Flüssig-Trennung bei ausreichend hoher Temperatur und Verdünnungsgrad, um das Alkalisalz der 3-Aminopyrazin-2-carbonsäure in Lösung zu halten, kann aus dem Filtrat wahlweise die 3-Aminopyrazin-2-carbonsäure mittels Mineralsäuren gefällt und abgetrennt werden, oder aber man läßt aus dem Filtrat, ggf. nach vorheriger Einengung und/oder Erhöhung der Kaliumionenkonzentration und/oder Zugabe eines organischen, mit Wasser mischbaren Lösemittels, durch Abkühlung das Alkalisalz der 3-Aminopyrazin-2-carbonsäure, vorzugsweise das Kaliumsalz, auskristallisieren und trennt es durch eine übliche Fest-Flüssig-Trennung ab. Das abgetrennte Pterin ist erneut als Ausgangsstoff verwendbar.

Sehr bevorzugt wird die genannte Fällung von nicht umgesetztem Pterin mittels eines Essigsäureesters oder ggf. einer Säure bei pH 3 bis 10 kombiniert mit Reaktionstemperaturen von 150 bis 170 °C und Mengen von 2 bis 3 Mol Alkalihydroxid je Mol/Pterin vorgenommen. Weiter sehr bevorzugt sind in dieser Kombination 1,5 bis 3 Gew.-Teile Wasser je Mol Pterin im Reaktionsansatz enthalten.

Sofern nach der beschriebenen Reinigungsoperation das Zielprodukt in Form des Alkalisalzes, vorzugsweise des Kaliumsalzes, gewonnen werden soll, ist es vorteilhaft, die Alkaliionenkonzentration der Lösung durch Zugabe geeigneter Alkaliverbindungen, vorzugsweise des Hydroxids, Chlorids oder Carbonats, zu erhöhen. Ein aus einer derartigen Lösung nach Erhöhung der Kaliumionenkonzentration und/oder Einengung und/oder Zugabe eines mit Wasser mischbaren organischen Lösemittels mit hoher Reinheit gewonnenes Kaliumsalz der 3-Aminopyrazin-2-carbonsäure kann vorteilhaft wieder in der beschriebenen Weise mit einem Kaliumionen enthaltenden Waschwasser und/oder einem wäßrig-verdünnten Alkohol gewaschen werden.

Unabhängig davon, ob das Zielprodukt in Form der 3-Aminopyrazin-2-carbonsäure oder eines Alkalisalzes gewonnen werden soll, kann die heiße Reaktionslösung vor oder nach einer ggf. vorgenommenen Abtrennung von nicht umgesetztem Pterin unter Bedingungen bezüglich Temperatur und Verdünnungsgrad, unter denen das Alkalisalz der 3-Aminopyrazin-2-carbonsäure gelöst vorliegt, einer Behandlung mit Aktivkohle unterworfen werden. Eine Aktivkohle-Behandlung ist auch/oder im Falle der ggf. in beschriebener Weise durchgeführten Reinigungsoperation möglich.

Obwohl die erfindungsgemäße Pterin-Spaltung mit wäßrigem Alkali vorzugsweise in rein wäßrigem Medium durchgeführt wird, ist die zusätzliche Verwendung organischer, mit Wasser mischbarer Lösemittel, beispielsweise von Ethylenglykol, nicht ausgeschlossen. Sofern das Zielprodukt in Form der Säure gewonnen wird, können zur Spaltung des Pterins auch Mischungen von Natronlauge und Kalilauge verwendet werden.

## Beipiel 1

16,3 g Pterin (0,1 Mol) wurden in 36 g Wasser mit 9,7 g NaOH (0,24 Mol) auf 180 °C erhitzt. Nach 2 h wurde mit 75 ml Wasser verdünnt und im Eisbad abgekühlt. Nach Filtration, Wäsche mit Methanol und Trocknung wurden 11,7 g Natriumsalz der 3-Aminopyrazin-2-carbonsäure als Rohprodukt gewonnen. Das Salz wurde in 100 g warmem Wasser aufgenommen, filtriert und das Filtrat mit Salzsäure angesäuert. Das abfiltrierte und getrocknete Produkt ergab 7,8 g 3-Aminopyrazin-2-carbonsäure entsprechend einer Ausbeute von 56 % d.Th. bezogen auf das eingesetzte Pterin.

## Beispiel 2

65,2 g Pterin (0,4 Mol), 58,8 g KOH (1,05 Mol) und 120 g Wasser wurden 4 h auf 200 °C erhitzt. Danach wurde zunächst auf 90 °C abgekühlt, filtriert und das Filtrat auf ein Gewicht von 195 g eingeengt. Die aus dem auf Raumtemperatur abgekühlten Filtrat ausgefallenen gelben Kristalle wurden abfiltriert, mit 3 Portionen von je 16 g einer 30 Gew.-%igen wäßrigen $K_2CO_3$-Lösung und anschließend mit 60 Gew.-%igem wäßrigen Methanol gewaschen. Das bei Raumtemperatur im Luftstrom getrocknete Produkt ergab 63,9 g (81,9 % d.Th.) Kaliumsalz der 3-Aminopyrazin-2-carbonsäure mit 1 Kristallwasser. Durch Nachtrocknen bei 110 °C wurden 58 g wasserfreies Kaliumsalz erhalten.

## Beispiel 3

Der in Beispiel 2 beschriebene Versuch wurde mit 56 g KOH (1,0 Mol) und einer Reaktionszeit von 6 h bei 180 °C wiederholt. Es wurden 52,2 g wasserfreies Kaliumsalz (73,7 % d.Th.) erhalten.

## Beispiel 4

Beispiel 2 wurde mit 65,2 g Pterin, 160 g Wasser und 50 g KOH (0,9 Mol) bei 190 °C und einer Reaktionszeit von 3 h wiederholt. Dem auf 90 °C abgekühlten Reaktionsgemisch wurden 36 g KOH zugesetzt, dann auf Raumtemperatur weiter abgekühlt und wie üblich aufgearbeitet. Es wurden 54 g wasserfreies Kaliumsalz (76,3 % d.Th.) erhalten. Das Produkt wurde in 450 g Wasser gelöst, die Lösung bei 40 °C mit 0,5 g Aktivkohle behandelt und filtriert. Das Filtrat wurde unter Rühren mit 25 g Essigsäuremethylester versetzt, wobei 1,7 g Pterin ausfielen und abfiltriert wurden. Das Filtrat wurde nach Zugabe von 40 g $K_2CO_3$ auf 180 g eingeengt und nach dem Abkühlen das Zielprodukt abfiltriert, mit $K_2CO_3$-Lösung und wäßrigem Methanol gewaschen und getrocknet. Es wurden 47,4 g (67 % d.Th.) Kaliumsalz der 3-Aminopyrazin-2-carbonsäure mit einer Reinheit von über 99,5 % erhalten.

## Beispiel 5

Beispiel 2 wurde mit 65,2 g Pterin, 67 g KOH (1,2 Mol), 175 g Wasser und 14 g Glykol im Einsatzgemisch bei 190 °C und 4 h Reaktionszeit wiederholt. Aus dem auf Raumtemperatur abgekühlten Reaktionsgemisch wurden 50,1 g (70,8 % d.Th.) Kaliumsalz der 3-Aminopyrazin-2-carbonsäure isoliert. Die Umkristallisation dieses Produktes entsprechend Beispiel 4 ergab keine Pterin-Fällung aus der mit Essigsäuremethylester versetzten wäßrigen Lösung. Das wie in Beispiel 4 beschrieben zurückgewonnene Produkt ergab 47,9 g (67,6 % d.Th.) mit einer Reinheit von über 99,5 %

## Beispiel 6

32,6 g Pterin (0,2 Mol) wurden mit 28 g KOH (0,5 Mol) in 128 g Wasser 13 h auf 160 °C erhitzt. Die auf 70 °C abgekühlte Reaktionslösung wurde mit Salzsäure auf einen pH-Wert von 2,9 gebracht, die dabei ausgefallene 3-Aminopyrazin-2-carbonsäure bei 5 °C abfiltriert und wieder in Kalilauge (12,3 g KOH, 200 g $H_2O$) gelöst. Diese Lösung wurde mit 23 g Methylacetat versetzt, aufgekocht und die dabei ausgefallenen 2,5 g Pterin (7,7 % vom Einsatz) abfiltriert. Das Filtrat wurde nach Zugabe von 5,6 g KOH und 27,6 g $K_2CO_3$ auf ein Gesamtgewicht von 135 g eingeengt und auf Raumtemperatur abgekühlt. Die ausgefallenen gelben Kristalle wurden abfiltriert, mit 20 g einer 30 %igen wäßrigen $K_2CO_3$-Lösung und 20 g 60 %igem Methanol gewaschen und bei 110 °C getrocknet. Es wurden 25 g Kaliumsalz der 3-Aminopyrazin-2-carbonsäure (0,14 Mol), entsprechend 76,5 % d.Th. bezogen auf umgesetztes Pterin, mit einer Reinheit von 99,5 % erhalten.

Beispiel 7

Die in Beispiel 6 beschriebene Umsetzung wurde mit nur 108 g Wasser im Einsatzgemisch wiederholt. Nach beendeter Reaktion wurde aus der Reaktionslösung unter Normaldruck $NH_3$ verkocht. Danach wurden 23 g Methylacetat zugesetzt und nochmals kurz aufgekocht. Während der pH-Wert auf 9 sank, fiel Pterin aus. Es wurden wiederum 2,5 g Pterin (7,7 % vom Einsatz) bei 60 °C abfiltriert. Aus dem auf 115 g eingeengten und auf Raumtemperatur abgekühlten Reaktionsgemisch wurden nach der in Beispiel 6 beschriebenen Aufarbeitung 25,7 g Kaliumsalz der 3-Aminopyrazin-2-carbonsäure (0,145 Mol), entsprechend 72,6 % d.Th. bezogen auf den Einsatz bzw. 78,6 % d.Th. bezogen auf den Umsatz, mit einer Einheit von 99,5 % erhalten.

**Ansprüche**

1. Verfahren zur Herstellung von 3-Aminopyrazin-2-carbonsäure, dadurch gekennzeichnet, daß 2-Amino-4-hydroxypteridin (Pterin) bei Temperaturen von 140 bis 220 °C mit gegebenenfalls zusätzliche organische Lösungsmittel enthaltender Natronlauge und/oder Kalilauge behandelt wird, wonach wahlweise durch Ansäuern der Reaktionslösung die 3-Aminopyrazin-2-carbonsäure gefällt und durch eine übliche Fest-Flüssig-Trennung isoliert wird oder aber aus der ggf. weiter eingeengten Reaktionslösung nach dem Abkühlen das Kalium- oder Natriumsalz der 3-Aminopyrazin-2-carbonsäure durch eine übliche Fest-Flüssig-Trennung abgetrennt wird.

2. Verfahren nach Anspruch 1, dadurch gekennzeichnet, daß pro Mol Pterin weniger als 4, vorzugsweise 2 bis 3 Mol Alkalihydroxid eingesetzt werden.

3. Verfahren nach Anspruch 1 oder 2, dadurch gekennzeichnet, daß im Einsatzgemisch pro Gew.-Teil Pterin 1,5 bis 3 Gew.-Teile Wasser eingebracht werden.

4. Verfahren nach einem der Ansprüche 1 bis 3, dadurch gekennzeichnet, daß die Umsetzung bei Temperaturen von 150 bis 170 °C durchgeführt wird.

5. Verfahren nach einem der Ansprüche 1 bis 4, dadurch gekennzeichnet, daß das Reaktionsgemisch vor Isolierung des Zielproduktes bei erhöhten Temperaturen, die ausreichen, um das Alkalisalz der 3-Aminopyrazin-2-carbonsäure in Lösung zu halten, auf einen pH-Wert von kleiner 10 und größer 3 gebracht und das dabei ausgefallene nicht umgesetzte Pterin abgetrennt wird.

6. Verfahren nach Anspruch 1 bis 4, dadurch gekennzeichnet, daß von dem aus dem Reaktionsgemisch isolierten Rohprodukt eine wäßrige Lösung eines Alkalisalzes der 3-Aminopyrazin-2-carbonsäure bereitet, diese bei erhöhten Temperaturen auf einen pH-Wert von 3 bis 10 gebracht, das dabei ausgefallene nicht umgesetzte Pterin abgetrennt und aus der Lösung mittels einer Mineralsäure die 3-Aminopyrazin-2-carbonsäure gefällt und abgetrennt oder aber das Alkalisalz der 3-Aminopyrazin-2-carbonsäure isoliert wird.

7. Verfahren nach Anspruch 5 oder 6, dadurch gekennzeichnet, daß zur Einstellung des pH-Wertes zwischen 3 und 10 in der wäßrigen Lösung des Alkalisalzes der 3-Aminopyrazin-2-carbonsäure eine Carbonsäure oder vorzugsweise ein Carbonsäureester, sehr bevorzugt Essigsäuremethyl oder -ethylester, verwendet wird.

8. Verfahren zur Gewinnung eines Alkalisalzes der 3-Aminopyrazin-2-carbonsäure, vorzugsweise des Kaliumsalzes, nach einem der Ansprüche 1 bis 7, dadurch gekennzeichnet, daß der wäßrigen Lösung des Alkalisalzes der 3-Aminopyrazin-2-carbonsäure eine entsprechende Alkaliverbindung, vorzugsweise als Hydroxid, Chlorid oder Carbonat, zugesetzt und die Lösung ggf. nach Einengung und/oder Zusatz eines organischen, mit Wasser mischbaren Lösemittels abgekühlt und das ausgefallene Alkalisalz der 3-Aminopyrazin-2-carbonsäure durch eine übliche Fest-Flüssig-Trennung abgetrennt wird.

## EINSCHLÄGIGE DOKUMENTE

| Kategorie | Kennzeichnung des Dokuments mit Angabe, soweit erforderlich. der maßgeblichen Teile | Betrifft Anspruch | KLASSIFIKATION DER ANMELDUNG (Int Cl⁵) |
|---|---|---|---|
| D,A | THE JOURNAL OF THE AMERICAN CHEMICAL SOCIETY, Band 67, Januar-Juni 1945 Easton, PA. USA J. WEIJLARD et al. "Some New Aminopyrazines and their Sulfanilamide Derivatives" Seiten 802-806 * Seite 804, linke Spalte, Zeile 63 – rechte Spalte, Zeile 6 * | 1 | C 07 D 241/28 |
| A | DE - A - 1 920 173 (MERCK) * Beispiele * | 1 | |
| D,A | DE - A1 - 3 723 874 (HÜLS) * Zusammenfassung * | 7,8 | |

RECHERCHIERTE SACHGEBIETE (Int Cl⁵)

C 07 D 241/00

Der vorliegende Recherchenbericht wurde für alle Patentansprüche erstellt.

| Recherchenort | Abschlußdatum der Recherche | Prüfer |
|---|---|---|
| WIEN | 07-01-1991 | LUX |